# EUROPEAN PATENT SPECIFICATION

(11) **EP 1 158 895 B1**
(45) Date of publication and mention of the grant of the patent: **18.02.2009**
(21) Application number: 00917774.2
(22) Date of filing: 07.03.2000
(51) Int. Cl.: A61B 5/00

(54) **METHOD AND CIRCUIT FOR STORING AND PROVIDING HISTORICAL PHYSIOLOGICAL DATA**
VERFAHREN UND SCHALTUNG ZUR SPEICHERUNG UND BEREITSTELLUNG VON HISTORISCHEN PHYSIOLOGISCHEN DATEN
PROCEDE ET CIRCUIT DE STOCKAGE ET DE RESTITUTION D'UN DOSSIER DE DONNEES PHYSIOLOGIQUES

(30) Priority: 08.03.1999 US 123266 P
(43) Date of publication of application: 05.12.2001
(73) Proprietor: Nellcor Puritan Bennett LLC, North Haven CT 06473 (US)
(72) Inventor: SWEDLOW, David, B., Danville, CA 94506 (US); DALEO, Stephen, L., Dublin, CA 94568 (US); YORKEY, Thomas, J., San Ramon, CA 94583 (US); RICHARDS, Edward, M., Pleasanton, CA 94566 (US); PORGES, Charles, Orinda, CA 94563 (US); STUART, Charles, San Jose, CA 95111 (US); NEMITS, Daniel, M., San Francisco, CA 94121 (US); DELONZOR, Russell, L., San Ramon, CA 94583 (US)
(74) Representative: Grubert, Andreas
(86) International application number: PCT/US2000/005892
(87) International publication number: WO 2000/053082

(56) References cited:
- EP-A- 0 504 935
- US-A- 3 925 762
- US-A- 4 827 943
- US-A- 5 518 001
- US-A- 5 758 644

## Description

### BACKGROUND OF THE INVENTION

The present invention relates to physiological test instruments and, in particular, sensors that include a mechanism for storing and providing to a monitor historical physiological data such as blood oxygen saturation data.

Pulse oximetry is typically used to measure various blood flow characteristics including, but not limited to, the blood oxygen saturation of hemoglobin in arterial blood, the volume of individual blood pulsation supplying a tissue, and the rate of blood pulsation corresponding to each heartbeat of a patient. Measurement of these characteristics has been accomplished by the use of a non-invasive sensor that passes light through a portion of a patient's blood perfused tissue and photo-electrically senses the absorption and scattering of light in such tissue. The amount of light absorbed is then used to estimate the amount of blood constituent in the tissue. The "pulse" in pulse oximetry comes from the time varying amount of arterial blood in the tissue during the cardiac cycle. The signal processed from the sensed optical signal is the familiar plethysmographic waveform due to cycling light attenuation.

To estimate blood oxygen saturation of a patient, conventional two-wavelength pulse oximeters emit light from two light emitting diodes (LEDs) into a pulsatile tissue bed and collect the transmitted light with a photodiode (or photo-detector) positioned on an opposite surface (i.e., for transmission pulse oximetry) or an adjacent surface (i.e., for reflectance pulse oximetry). One of the two LEDs' primary wavelength is selected at a point in the electromagnetic spectrum where the absorption of oxyhemoglobin (HbO₂) differs from the absorption of reduced hemoglobin (Hb). The second of the two LEDs' wavelength is selected at a different point in the spectrum where the absorption of Hb and Hb O₂ also differs from each other, and further differs from those at the first wavelength. Commercial pulse oximeters typically utilize one wavelength in the near red part of the visible spectrum near 660 nanometers (nm) and one in the near infrared (IR) part of the spectrum in the range of 880-940 nm.

Oxygen saturation can be estimated using various techniques. In one common technique, the photo-current generated by the photo-detector is conditioned and processed to determine the modulation ratio of the red to infrared signals. This modulation ratio has been observed to correlate well to arterial oxygen saturation. The pulse oximeters and sensors are empirically calibrated by measuring the modulation ratio over a ranee of in vivo measured arterial oxygen saturations (SaO₂) on a set of patients, healthy volunteers, or animals. The observed correlation is used in an inverse manner to estimate blood oxygen saturation (SpO₂) based on the measured value of modulation ratios of a patient. The estimation of oxygen saturation using modulation ratio is described in U.S. Patent No. 5,853,364, entitled "METHOD AND APPARATUS FOR ESTIMATING PHYSIOLOGICAL PARAMETERS USING MODEL-BASED ADAPTIVE FILTERING", issued December 29, 1998, and U.S. Patent No. 4,911,167, entitled "METHOD AND APPARATUS FOR DETECTING OPTICAL PULSES", issued March 27, 1990. The relationship between oxygen saturation and modulation ratio is further described in U.S. Patent No. 5,645,059, entitled "MEDICAL SENSOR WITH MODULATED ENCODING SCHEME," issued July 8, 1997. All three patents are assigned to the assignee of the present invention.

The LEDs and photo-detector are typically housed in a reusable or disposable oximeter sensor that couples to the pulse oximeter electronics and the display unit (hereinafter referred to as the monitor). The sensors are often connected to patients for long periods of time. Conventionally, historical physiological data for the patient is collected, if at all, by the monitor coupled to the, sensor. The historical data can be valuable to a clinician or medical personnel for diagnostic and monitoring purposes.

Patients are often moved to various locations during treatment For example, a patient may be picked up in an ambulance, delivered to an emergency room, moved to an operating room, transferred to a surgical recovery room, transferred to an intensive care unit, and then moved to a nursing floor or other locations. Thus, the patient may be moved between various locations within the same hospital, or between different hospitals. In many instances, the sensor employed to monitor the conditions of the patient is adhesive in their attachment and therefore remains with the patient. The monitors, however, are typically local to particular locations within the facility. The sensor is normally disconnected from the monitor at the departure site and reconnected to another monitor at the destination site. Consequently, any historical physiological data collected by the monitor at the departure site is normally unavailable to the clinician attending the patient at the destination site.

In the medical art, a combination of a catheter sensor and a memory unit is disclosed in U.S. Patent No. 4,858,615, entitled "CATHETER SENSOR AND MEMORY UNIT," and issued August 22, 1989. In this patent, the sensor assembly (34) is located at a distal end of the catheter (32) and the memory unit (38) is connected by a multi-conductor lead (40) to the sensor (see Fig. 5). The catheter is an invasive instrument typically used at a particular location and removed during transport. Neither the catheter nor the memory unit would travel with the patient as he or she is moved to different locations. Thus, any data captured and stored in the memory unit (38) is also not available when the catheter is removed from the patient.

Accordingly, it is highly desirable to provide mechanisms for storing and providing historical physiological data that travels with a patient.

### SUMMARY OF THE INVENTION

The invention provides a mechanism for storing and providing historical physiological data, such as blood oxygen saturation data, for a patient, as defined in claim 1. In particular, the historical physiological data is stored in a storage medium that "travels" with the patient and is accessible wherever the patient is moved. This is achieved by storing the physiological data within the sensor assembly. At the destination site, a monitor or a device capable of interfacing with the sensor electronics can retrieve and display the data. The historical physiological data allows a clinician or medical personnel at the destination site to assess the condition of the patient for the entire time that the patient has been monitored. The invention can be used to store and provide various types of physiological data including, but not limited to, blood oxygen saturation, heart rate, and temperature data.

A specific embodiment of the invention provides a physiological sensor that includes a number of light sources, at least one photo-detector, and a memory circuit. The light sources are selected to operate at different wavelengths. The photo-detector receives light emitted by the plurality of light sources. And the memory circuit stores physiological data and provides the data when requested. The physiological data is indicative of a physiological condition of a patient being monitored by the sensor.

Another specific embodiment of the invention provides a physiological test instrument that includes a monitor and a sensor according to claim 20. The monitor includes conditioning circuitry and processing circuitry. The conditioning circuitry receives an electrical signal and processes the electrical signal to provide sampled data. The processing circuitry processes the sampled data to provide physiological data, wherein the physiological data is indicative of a physiological condition of a patient. The sensor couples to the monitor and includes a number of light sources, at least one photo-detector, and a memory circuit. The light sources are selected to operate at different wavelengths. The photo-detector receives light emitted by the light sources. The memory circuit stores the physiological data and provides the data when requested. An encoder can optionally be coupled to the processing circuitry to code and compress the physiological data before storage to the memory circuit. The test instrument can be an oximeter system for storing and providing historical saturation data of a patient.

Yet another specific embodiment of the invention provides a method for storing physiological data, as defined in claim 15. The method detects, via a sensor, at least one signal indicative of a physiological condition and conditions the detected signal to generate data samples. The data samples are processed to generate the physiological data, wherein the physiological data describes the physiological condition. The physiological data is stored within a memory located within the sensor. The physiological data can be coded and compressed before storage to the memory.

The foregoing, together with other aspects of this invention, will become more apparent when referring to the following specification, claims, and accompanying drawings.

### BRIEF DESCRIPTION OF THE DRAWINGS

Fig. 1 shows a simplified block diagram of an embodiment of a physiological measurement system;
Fig. 2 shows a block diagram of an embodiment of a monitor and a sensor; and
Fig. 3 shows a block diagram of one compression scheme for oxygen saturation data.

### DESCRIPTION OF THE SPECIFIC EMBODIMENTS

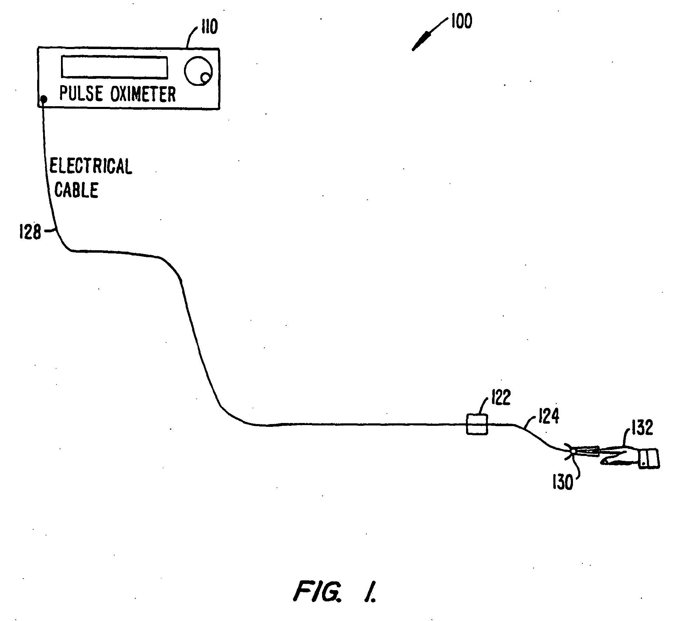

Fig. 1 shows a simplified block diagram of an embodiment of a physiological measurement system 100. System 100 includes a monitor 110 that couples to a display unit 120 via an electrical cable 122. Monitor 110 further couples via a second electrical cable 128 to a sensor 130 that is applied to a patient 132. Sensor 130 includes light sources (e.g., LEDs) and a photo-detector along with suitable components to couple the electro-optical components to electrical cable 128. Sensor 130 is shown in Fig. 1 as a clip-on sensor. However, the invention can be applied to many sensor implementations, including those attached to a patient by adhesive and other attachment means. In a specific embodiment, monitor 110 is a pulse oximeter.

For estimating blood oxygen saturation, light from light sources at two or more wavelengths (e.g., red and infrared) is transmitted through a patient's blood perfused tissues (e.g., in a finger) and detected by a photo-detector. The selection of the wavelengths is based on a number of factors. Such factors include the absorption characteristics of the patient and transmission medium. The light sources and photo-detector are typically housed within a sensor that couples to the monitor (e.g., the pulse oximeter). The detected optical signal is then provided to the monitor for processing.

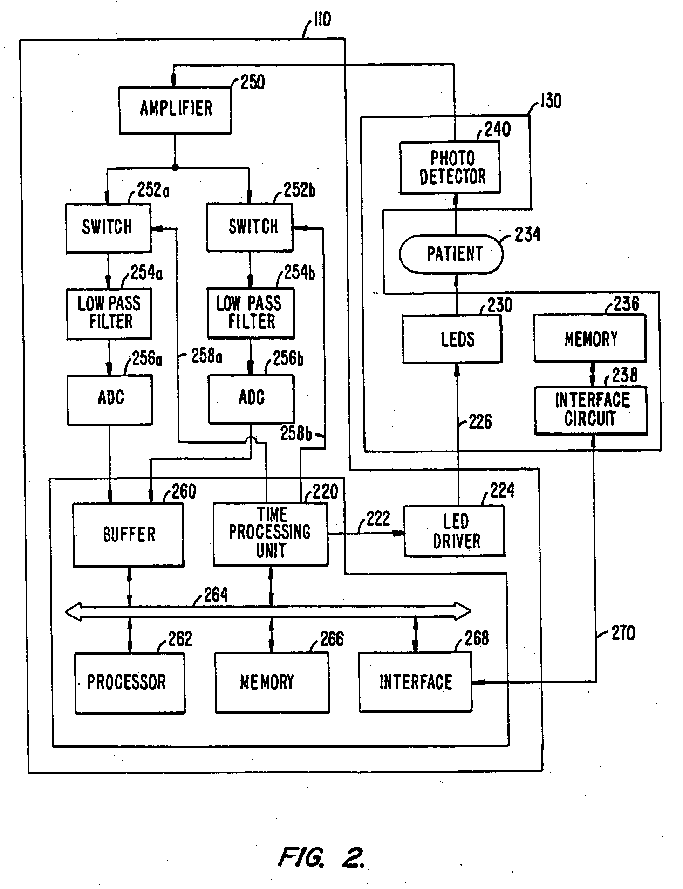

Fig. 2 shows a block diagram of an embodiment of monitor 110 and sensor 130. Within monitor 110, a time processing unit (TPU) 220 provides control signals 222 to an LED driver 224 that, via data line(s) 226, alternately activates LEDs 230 within sensor 130. Depending on the particular implementation, LEDs 230 include two or more LEDs and LED driver 224 provides the necessary drive signals for the LEDs. When activated, the light from LEDs 230 passes through a medium (e.g., air or a fiber optic cable, depending on the implementation) into a patient's tissues 234. After being transmitted through or reflected from the tissues, the light is received by a photo-detector 240 via another medium (e.g., air or another fiber optic cable). Photo-detector 240 converts the received light into a photo-current, which is then provided to an amplifier 250 that amplifies the photo-current.

As shown in Fig. 2, the amplified signal from amplifier 250 is provided to circuitry for two different channels, one channel for each of the red and infrared wavelengths. For a three-wavelength implementation, circuitry is provided for three channels. Each channel circuitry includes an analog switch 252 coupled in series with a low pass filter 254 that is further coupled in series with an analog-to-digital converter (ADC) 256. Control lines 258 from time processing unit 220 select the sampled data from the channel corresponding to the LED being activated. Specifically, the sampled data from ADC 256a is selected when the red LED is activated and the sampled data from ADC 256b is selected when the infrared LED is activated. The sampled data from ADCs 256 is provided to a buffer 260 that stores the data for further processing. In an implementation, as buffer 260 periodically fills up, a processor 262 coupled to a bus 264 directs the transfer of the data from buffer 260 into a memory 266. The monitor implementation shown in Fig. 2 is one of many implementations. Another pulse oximeter implementation is disclosed in the aforementioned U.S. Patent No. 5,853,364. The present invention can be adapted for application in various monitor implementations.

The sensor of the invention further includes circuitry that stores historical physiological data and provides the data when requested. As shown in Fig. 2, sensor 130 includes a memory 236 coupled to an interface circuit 238. Interface circuit 238 provides signal conditioning, and can also provide other functions such as address decoding, and so on. Interface circuit 238 couples via a bus 270 to a data interface circuit 268 within monitor 110. Through interface circuits 238 and 268, physiological data is transferred between monitor 110 and sensor 130.

In an embodiment, to enhance compatibility of the sensor of the invention with conventional sensors and conventional monitors, bus 270 is implemented using new signal lines (i.e., not using or sharing the existing signal lines of conventional sensors). Bus 270 can be implemented as a serial bus, a parallel bus, or other bus architectures. With this implementation, when sensor 130 of the invention is plugged into a monitor not capable of supporting the features of the invention, the signals on interface circuit 238 are simply ignored by the monitor, or alternatively not requested by the monitor.

In another embodiment, interface circuits 238 and 268 interact via signal line(s) or wire(s) existing in conventional sensors and monitors. For example, interface circuits 238 and 268 can couple via data line(s) 226 and time multiplex with the LED drive signals from LED driver 224.

Time processing unit 220, buffer 260, processor 262, memory 266, and data interface circuit 268 can be implemented in various manners. For example, these elements can be implemented within a single integrated circuit, such as a DMC68HC16 micro-controller from Motorola. These elements can also be implemented within an application specific integrated circuit (ASIC), a digital signal processor, a micro-controller, or other circuits.

Memory 236 can be implemented as a random access memory (RAM), a FLASH memory, a programmable read only memory (PROM), an erasable PROM (EPROM), an electrically erasable PROM (EEPROM), a similar programmable and/or erasable memory, any kind of erasable memory, a write once memory, or other memory technologies capable of write operations. Memory 236 and interface circuit 238 can be integrated within one integrated circuit for reduced size and cost.

In a specific embodiment, to preserve the historical data and prevent accidental erasure, the sensor memory can be written once. This memory characteristic also prevents erasure of the data during sensor processing. A specific example of a memory device that can be written once is a 2-wire EPROM device available from Dallas Semiconductor Corp.

In another embodiment, the memory can be erased and overwritten multiple times. This memory characteristic may be advantageous, for example, for non-disposable sensors that may include a large amount of memory. Such "specialty" sensors may be better suited for applications where there is a higher propensity to use reusable sensors, such as inside an operating room or an intensive care unit or during an ambulance transport. Specific examples of memory devices that can be erased and overwritten are Flash, EEPOM, battery backed RAM, and other technologies.

The invention is applicable for various oximeter system implementations. For example, in an embodiment, an adapter module and a fiber optic cable can be interposed between cable 128 and sensor 130 (see Fig. 1). The adapter module can include the light sources, the detector, and suitable optics to couple the electro-optical components to the fiber optic cable that guides light to and receives light from the patient. The fiber optic cable can also be partitioned into a long extension cable and a relatively short "sensor" cable. The fiber optic cables can be either glass or plastic fiber. This embodiment allows the electro-optical components to be reused, and only the short sensor cable is replaced from patient to patient.

Fig. 2 shows an oximeter implementation using light at two wavelengths. However, light from more than two LEDs can be used (i.e., for improved accuracy). Light from a single light source can also be used, typically along with appropriate optical filter. Moreover, light sources other than LEDs can be used. For example, lasers or white light sources can be used with appropriate filters at either the transmitting or receiving end.

The sensor can include different numbers of elements, depending on the implementation of the sensor or the application for which the sensor is used. In one implementation, the sensor includes the LEDs and the photo-detector. This implementation reduces the transmission loss by placing the light source and the detector near the patient. In another implementation, the sensor includes only the transmission medium (e.g., a short fiber optic cable), but no LEDs or photo-detector. This implementation reduces cost, since the LEDs and photo-detector are included within an adapter module and are reusable. In yet another implementation, the sensor can include either the LEDs or the photo-detector, as a compromise to reduce cost and transmission loss. For these various variations, the sensor includes the memory for storing historical physiological data.

During normal operation, when the sensor is plugged into the monitor, the monitor receives the signal from the photo-detector within the sensor and processes this signal to obtain the desired physiological data. In some conventional monitors, the physiological data is stored in a memory within the monitor and retrieved at a later time when requested. However, when a patient is moved to new locations and different monitors are used, the data stored in the monitor at the previous site is typically not available at the current site.

In accordance with the invention, the physiological data is processed, displayed, and stored in the monitor in the nominal manner. In addition, the data is compressed and provided to the sensor for storage in a memory 236 located within the sensor. When the sensor is plugged into another monitor, the new monitor can retrieve the data stored in the sensor memory, decompress the retrieved data, and display the decompressed data. In an embodiment, when the sensor is first plugged into a new monitor, the monitor retrieves and displays the historical physiological data for the most recent predetermined period (i.e., the last 20 or 30 minutes). This predetermined period can be programmed by the clinician or can be preprogrammed into the sensor memory.

Alternatively, the monitor can be configured to retrieve and display the historical physiological data at any time upon request by a health care giver (or a clinician), by the health care giver simply activating a control knob on the monitor. The control knob optionally can be preset so as to automatically retrieve the data upon occurrence of a predetermined event, such as a sensor being plugged into the monitor, or can by preconfigured so that the data is only retrieved upon explicit command by a health care giver.

As noted above, the invention can be used to store and provide various physiological data including, but not limited to, blood oxygen saturation and heart rate data. For clarity, the invention is described in the context of the storage and retrieval of blood oxygen saturation (SpO₂) data. Based on the received signals representative of the intensity of the light detected by photo-detector 240, processor 262 estimates oxygen saturation using algorithms that are known in the art. These algorithms utilize calibration coefficients that may be empirically determined and correspond to, for example, the wavelengths of the lights used.

The saturation data for a particular patient is processed by the monitor attached to the sensor, and the processed data is provided to the sensor for storage in the sensor memory. The selection of the sensor memory is dependent on numerous factors including cost, the amount of data that needs to be stored for a particular application, the amount of achievable data compression, the physical dimensions, and so on. For oxygen saturation, storage of approximately seven days of historical data is adequate for many applications.

In an embodiment, to reduce the amount of data to be stored in the sensor memory, the physiological data is compressed before storage. In an embodiment, the compression is performed by facilities located within the monitor. Alternatively, the encoding circuit can be on the sensor itself. The monitor further includes facilities to decompress the data retrieved from the sensor memory. Compression allows for the use of a smaller-sized memory in the sensor. This is particularly advantageous since the sensor is typically disposed after use on a patient. Compression also allows more data to be stored into a memory of a given size. The ability to store large amount of data is important for many diagnostic applications that require data collected over hours or days.

The compression scheme can be designed to take advantage of known characteristics of the physiological data being stored. For example, it is known that oxygen saturation generally do not change rapidly. This characteristic can be exploited to achieve significant compression, as described below.

Fig. 3 shows a block diagram of one compression scheme for oxygen saturation data. The saturation data is provided to a filter 312 that filters the data. The filtered data is provided to a differential pulse code encoder (DPCM) 314 that determines difference values between successive filtered data samples. The difference data is provided to a quantizer 316 that "re-quantizes" the difference data. The quantized data is provided to a run-length coder (RLC) 318 that codes the quantized data using an efficient set of codes. Each of these elements is further described below.

In an embodiment, since it is known that oxygen saturation does not change rapidly, the saturation data is averaged over a predetermined time period (herein referred to as an epoch) and one averaged saturation sample is provided as representative of the saturation during that epoch. In a specific embodiment, an epoch is a time period having a duration of one to five minutes, although any different duration can be used. The epoch can also be set based on the characteristics of the physiological data being stored (i.e., a longer epoch for slow changing physiological data and a shorter epoch for fast changing data).

Filter 312 filters the saturation data. Filter 312 can be a digital filter designed in a manner known in the art. In an embodiment, filter 312 is a lowpass filter having a bandwidth related to the epoch (i.e., BW ≈ α/ t_{EPOCH}, where BW is the filter bandwidth, α is a proportionality constant, and t_{EPOCH} is the period of an epoch. The characteristics of filter 312 can also be equalized (i.e., spectrally shaped) to match the characteristics of the data being filtered.

To further smooth the data and increase the amount of compression, the saturation data can be filtered over a period of several epochs. However, averaging the saturation data over a longer time interval masks rapid changes in saturation, which are smoothed out and lost in the averaging process. To capture rapid change events, a moving average filter can be used.

In an embodiment, the moving average filter includes a filter that filters the saturation data over an epoch (i.e., a single-epoch filter) and another filter that filters the data over multiple epochs (i.e., a multiple-epoch filter). The moving average filter monitors the averaged saturation data from the single-epoch filter and detects averaged saturation samples that fall outside a predetermined window. The predetermined window can be set at plus and minus several saturation points around the current averaged saturation value. For example, if the current averaged saturation sample has a value of 90 saturation points, the predetermined window can be set at ±2 saturation points centered around 90 (e.g., 88 to 92). The moving average filter then activates a flag if the next averaged saturation sample has a value below 88 or greater than 92. If the averaged saturation sample from the single-epoch filter is within the window, the averaged sample from the multiple-epoch filter is used. Otherwise, an averaged saturation sample from the single-epoch filter falling outside the window indicates a rapid change in saturation. This detected sample is used to restart the moving average and cause a change of the averaged saturation sample to the new value from the single-epoch filter. The moving average filter allows for the detection of rapid changes and the capture of their magnitudes while maintaining a filtered data stream that enhances compression of nominal data.

The slow varying nature of oxygen saturation suggests the use of differential coding since less bits would be required to represent the differences between samples than the actual sample values. With differential coding, the first saturation sample is stored using the actual sample value. A subsequent saturation sample is represented as a delta value from a preceding saturation sample. Periodically, the actual sample value is stored to prevent an accumulation of error in the differential coding and to limit the propagation of error. DPCM 314 determines the difference values between successive saturation samples. The difference value is calculated by subtracting the current saturation sample from a preceding saturation sample.

For many applications, it is not necessary to store saturation data with a great deal of precision. For example, for some applications, it is sufficient and acceptable to indicate a change of ± one saturation point as no change in saturation. Thus, the difference values from DPCM 314 can be re-quantized by quantizer 316.

In an embodiment, quantizer 316 is a window comparator having a quantization window of, for example, ± one saturation point. If the difference value falls within the quantization window, quantizer 316 indicates a "no change" in saturation and outputs a zero. If the difference value falls outside the quantization window, quantizer 316 passes this value without additional processing. Quantizer 316 can also be implemented in other manners, for example, as a quantizer having a step size twice that of the saturation sample.

Requantization by quantizer 316 introduces quantization error in the reconstructed data. This error can accumulate over successive samples and exceed an acceptable threshold. To avoid this phenomenon, an error accumulator 320 coupled to quantizer 316 accumulates the error introduced by quantizer 316 and provides the accumulated error to DPCM 314. DPCM 314 takes the accumulated error into account when calculating the difference values.

Because of the slow varying nature of oxygen saturation and the use of differential coding and requantization, many of the data values from quantizer 316 are zero. In an embodiment, run length coder (RLC) 318 receives the quantized data from quantizer 316, transmits the non-zero values, and sends a code representative of the number of zero values between the non-zero values. For example, for a sequence of (3, 0, 0, 0, 0, 0, 0, 4, ...), RLC 318 transmits the first "3", then a code indicating six consecutive zeros, then "4". In an embodiment, the code representative of the number of consecutive zeros are generated such that the most likely sequences of consecutive zeros are assigned codes having shorter code widths, and the more unlikely sequences are assigned codes having longer code widths. This code characteristic is similar to that of a Huffman code that is known in the art.

The elements shown in Fig. 3 can be implemented in various manners. For example, these elements can be implemented within a processor (i.e., processor 262 in Fig. 2), a digital signal processor, an ASIC, or other circuits. The functions of the elements in Fig. 3 can also be provided by a program code executed on processor 262 with the supported of memory 266.

Fig. 3 shows one compression embodiment. In another compression embodiment, the non-zero difference values are transmitted along with their epoch numbers. For the sequence shown above, the transmitted values may be (3, 1), (4, 8), and so on. The first number in the pair is the difference value and the second number is the epoch number. For some applications, this embodiment may provide additional compression over the embodiment shown in Fig. 3.

In yet another compression embodiment, the saturation value and the number of epochs over which the value is within a predetermined quantization window are recorded. In this embodiment, it is not necessary to compute the difference values. Again, this embodiment may significantly reduce the data storage requirement for some type of physiological data.

Several compression embodiments have been described for oxygen saturation data. Although the invention can be practiced without the use of compression, additional capabilities are provided by the judicious use of compression. As used herein, compression includes any processing that alters, however slightly, the original form of the physiological data as they are generated (in the nominal manner) by the monitor. Other compression schemes can also be used and are within the scope of the invention. Of course, no compression could optionally be used.

Additional data besides oxygen saturation data can be stored in the sensor memory (i.e., to assist in diagnostics or monitoring of patients). For example, a time stamp of the data can be stored. In this case, the first data sample includes the specific time (e.g., date and time) when the data is recorded. Subsequent data samples can be indicated by the number of epochs away from the first (or a previous) data sample. The sensor memory can also store an indication of a disconnection of the sensor from the monitor. This data allows the clinician or medical personnel to delineate the events retrieved from the sensor memory.

The sensor memory can also include a field that indicates when the sensor memory is full. The information in this field can be provided to the monitor to direct the monitor to cease sending data to the sensor memory. The information in this field can be prominently displayed by the monitor to notify the clinician or medical personnel. Also, in response, the monitor can generate an alarm (i.e., blinking light or an audio alarm, or both) to draw the attention of the clinician to the operating state of the sensor.

In a specific embodiment, the saturation data is stored in a data format that includes an N-bit data field and a field containing the number of epochs over which the data value is maintained. However, many other data formats can be used and are within the scope of the invention.

As noted above, in a specific embodiment, the sensor memory is implemented as a write-once memory device. A field in the sensor memory can be set when the sensor is reprocessed so that the monitor can determine that it is coupled to a reprocessed sensor. The monitor can use the information in this field to disable the display of the historical data (for example, if the memory is write once and relatively full). Alternatively, if the memory is erasable, a field for storing historical physiological data could be erased during sensor reprocessing.

Disabling the data display may be preferable in some applications to ensure the integrity of the collected data. For a memory device that can be written once and has a fixed memory size, it may not be possible to determine where the "old" data came from or how much memory may still be available on a reprocessed sensor. Moreover, it is highly desirable to avoid having data from an old patient being displayed and potentially mistaken as valid data for the patient to which the sensor couples. Since it is not easy to control or determine the amount of available unwritten memory after a use, which can vary from zero to the full amount, inconsistency and potential customer dissatisfaction may result from using a sensor having widely varying amounts of available memory. By not displaying data from reprocessed sensors, these potential problems are avoided.

The invention has been described for the storage of blood oxygen saturation data. However, the sensor memory can also store data for other physiological characteristics such as, for example, heart beat, temperature, and so on. For example, anything, the sensor memory can be used to store NTBP, IBP, and ECG waveforms. Moreover, as memory costs continue to fall and larger memories become available, more complex physiological parameters can be measured and stored.

Additionally, information about the monitor can be stored or embedded along with the physiological data. This additional information may include, for example, the serial number of the monitor to which the sensor couples, the sensor connect/disconnect times, monitor diagnostics, and others. This information would allow the clinician access to historical information on the instrument as well as the physiological data, which might be useful, for example, in litigation or in troubleshooting and instrument.

The invention provides advantages not available in conventional monitors and sensors. For example, the invention allows for monitoring of a patient in transit who may be connected to two or more monitors over a period of time. One such situation is a patient who is transported in an ambulance to an emergency room and later transferred to an intensive care unit. The invention is especially beneficial in this application since this particular patient is more likely to be in need of close monitoring.

The invention can also be used to document physiological characteristics. For example, for a patient in home care who requires oxygen, documentation of oxygen saturation is typically needed. In this case, the sensor of the invention can be used to store saturation data for the patient over a predetermined time period (i.e., one week). At the end of this period, the caregiver can simply remove the sensor and sends it away as documentation of the patient's saturation. The invention can also be used to collect data for other applications such as, for example, sleep diagnostics, de-saturation, and so on.

The sensor of the invention has been described for use in combination with a monitor that performs the signal processing of the detected signal and compression of the processed data. In another embodiment, the sensor of the invention includes the facility to process (and compress, if necessary or desirable) the detected signal. This embodiment advantageously allows for independent operation of the sensor without support from a monitor. The data stored within the sensor can be provided to a monitor for display. The amount of signal processing and compression that can be achieved by circuitry within the sensor is only limited by the available technology, which inevitably improves over time. In the near term, physiological data that does not require extensive signal processing and compression (e.g., temperature, peak amplitude in a waveform, heart rate, and so on) can be collected and stored by the sensor.

For further understanding of the invention in its use for the storage of blood oxygen saturation data, a description of the derivation of oxygen saturation from photo-detected signals is included in the aforementioned U.S. Patent Nos. 4,911,167, 5,645,059, and 5,853,364.

The data stored can correspond to a value of an actual physiological condition (i.e., oxygen saturation) or can be indicative of the condition value with the condition value being determinable by the monitor upon reference to a look-up table or by the monitor calculating the condition value from the stored data using a predetermined algorithm.

The previous description of the preferred embodiments is provided to enable any person skilled in the art to make or use the present invention. The various modifications to these embodiments will be readily apparent to those skilled in the art, and the generic principles defined herein may be applied to other embodiments without the use of the inventive faculty. For example, the invention can be applied to the storage of other physiological data, such as data for a patient's heartbeat, temperature, volume of individual blood pulsation supplying the tissue or the rate of blood pulsation, and so on. Thus, the present invention is not intended to be limited to the embodiments shown herein but is to be accorded the widest scope consistent with the principles and novel features disclosed herein.

## Claims

1. A physiological sensor connectable to a remote monitor (110), the physiological sensor (130) comprising:
means (240) for obtaining signals from a patient (234) indicative of a physiological condition of the patient;
means (240) for sending the signals to the remote monitor;
a memory circuit (236) integrated within the sensor and located separately from the remote monitor; and
an interface circuit (238) coupled to the memory circuit (236), wherein the interface circuit (238) facilitates data transfer to and from the memory circuit (236),
**characterized in that** the memory circuit (236) is separate within the sensor and electrically isolated from the means (240) for obtaining signals and the means (240) for sending the signals; and adapted for transfer of patient physiological data derived from the signals and indicative of the physiological condition between the remote monitor (110) and the memory circuit (236) when requested by the remote monitor (110).

2. The sensor of claim 1, wherein the interface (238) can be coupled with the remote monitor (110) through separate signal lines (270).

3. The sensor according to one of the preceding claims, wherein the memory circuit (236) is implemented as a write-once memory, a FLASH memory, a random access memory (RAM), an erasable memory, or an electrically erasable programmable read only memory (EEPROM).

4. The sensor according to one of the preceding claims, wherein the memory circuit (236) is implemented as a multiple writes memory.

5. The sensor according to one of the preceding claims, wherein the physiological data includes blood oxygen saturation data.

6. The sensor of claim 5, wherein the physiological data includes pulse rate data.

7. The sensor according to one of the preceding claims, wherein the physiological data is compressed before storage to the memory circuit (236).

8. The sensor of claim 7, wherein the physiological data is compressed using either differential coding or run length coding.

9. The sensor of claim 7, wherein the physiological data is uncompressed when stored on the memory circuit.

10. The sensor according to one of the preceding claims, wherein the physiological data is subsampled to provide one data sample for each epoch, wherein an epoch is a predetermined time period selected, in part, based on characteristics of physiological data being stored.

11. The sensor according to one of the preceding claims, wherein the memory circuit (236) provides information that indicates when the memory circuit (236) is full.

12. The sensor according to one of the preceding claims, wherein the memory circuit (236) further stores information of a time associated with each of specific samples of the physiological data.

13. The sensor according to one of the preceding claims, wherein the memory circuit (236) further stores information indicative of disconnection of the sensor from a monitor (110).

14. The sensor according to one of the preceding claims, further comprising:
at least one light source (230), each light source is selected to operate at a different wavelength;
at least one photo-detector (240) operative to receive light emitted by the at least one light source; and
an interface circuit (238) coupled to the memory circuit, wherein the interface circuit coordinates data transfer to and from the memory circuit.

15. A method for storing physiological data comprising:
detecting via means (240) of a physiological sensor (130) according to any one of the preceding claims at least one signal indicative of a physiological condition;
conditioning the detected at least one signal to generate data samples;
processing the data samples in a remote monitor (110) separate from the sensor (130) to generate physiological data, wherein the physiological data describes the physiological condition; and
storing the physiological data within the memory (236) located separate within the sensor (130) from the means (240) and separate from the remote monitor (110).

16. The method of claim 15, wherein data transfer between the memory (236) and the remote monitor (110) is performed through separate signal lines (270).

17. The method according to one of the preceding claims 16 or 15, further comprising; coding the physiological data to generate compressed physiological data.

18. The method according to one of the preceding claims 15-17, further comprising; sampling or preprocessing the physiological data to generate compressed physiological data.

19. The method according to one of the preceding claims 15-18, wherein the physiological data includes blood oxygen saturation data.

20. A physiological test instrument (100) comprising:
a remote monitor (110) comprising:
- conditioning circuitry (250, 252, 254, 256) for receiving an electrical signal and processes the electrical signal to provide sampled data; and
- processing circuitry (262) for processing the sampled data to provide physiological data, wherein the physiological data is indicative of a physiological condition of a patient;
and
a physicological sensor (130) connectable to the remote monitor (110), wherein the physicological sensor (130) is a physicological sensor according to any of the claims 1-16, wherein the means (240) for obtaining signals comprises:
- at least one light sources (230), each light source is selected to operate at a different wavelength; and
- at least one photo-detector (240) operative to receive light emitted by the at least one light source (230).

21. The test instrument of claim 20, wherein the remote monitor (110) further includes means (268) responsive to a user input for transferring the at least some of the physiological data to the memory circuit (236) in response to user input.

22. The test instrument according to claim 20 or 21, wherein the remote monitor (110) further includes means (268) responsive to an oxygen desaturation event for transferring the at least some of the physiological data to the memory circuit (236) in response to an oxygen desaturation event of the patient.

23. The test instrument according to one of the preceding claims 20-22; wherein the remote monitor (110) further includes means (268) responsive to a threshold crossing for transferring the at least some of the physiological data to the memory circuit when an oxygen saturation of the patient differs by more than a predetermined amount from a previous oxygen saturation of the patient.

24. The test instrument according to one of the preceding claims 20-23, wherein the remote monitor (110) further includes an encoder (262) coupled to the processing circuitry, wherein the encoder codes the physiological data to provide compressed physiological data.

25. The test instrument of claim 24, wherein the monitor (110) further includes a decoder (262) that receives compressed physiological data from the memory circuit and decodes the data.

26. The test instrument according to one of the preceding claims 20-25, wherein the physiological data includes blood oxygen saturation data.

27. The test instrument according to one of the preceding claims 20-26, wherein the test instrument is an oximeter system for storing and providing historical saturation data of a patient comprising:
at least two light sources (230) for transmitting light through the patient, wherein the light sources operate at different wavelengths; in which the photo-detector is adapted to receive optical signals from the light sources and adapted to provide electrical signals indicative of received optical signals; wherein the physiological data produced by the processing circuitry is saturation data; and further comprising circuitry that directs display of the saturation data.

## Patentansprüche

1. Physiologischer Sensor, der an einen Fernmonitor (110) angeschlossen werden kann, wobei der physiologische Sensor (130) aufweist:
Mittel (240) zum Erhalt von Signalen eines Patienten (234), die den physiologischen Zustand des Patienten anzeigen;
Mittel (240) zum Senden der Signale an den Fernmonitor;
eine in den Sensor integrierte Speicherschaltung (236), die separat von dem Fernmonitor angeordnet ist; und eine an die Speicherschaltung (236) gekoppelte Schnittstellenschaltung (238), wobei die Schnittstellenschaltung (238) die Datenübertragung von und zu der Speicherschaltung (236) erleichtert,
**dadurch gekennzeichnet, dass** die Speicherschaltung (236) innerhalb des Sensors separat liegt und elektrisch von den Mitteln (240) zum Erhalt von Signalen und von den Mitteln (240) zum Senden der Signale isoliert ist; und die angepasst ist, die physiologischen Patientendaten, die von den Signalen abgeleitet werden und den physiologischen Zustand anzeigen, zwischen dem Fernmonitor (110) und der Speicherschaltung (236) zu übertragen, wenn diese vom Fernmonitor (110) angefordert werden.

2. Sensor nach Anspruch 1, wobei die Schnittstelle (238) mit dem Fernmonitor (110) durch separate Signalleitungen (270) gekoppelt werden kann.

3. Sensor nach einem der vorhergehenden Ansprüche, wobei die Speicherschaltung (236) als einmalig beschreibbarer Speicher, FLASH-Speicher, Speicher mit wahlfreiem Zugriff (RAM - random access memory), löschbarer Speicher oder elektrisch löschbarer, programmierbarer Festwertspeicher (EEPROM - electrically erasable programmable read only memory) ausgeführt ist.

4. Sensor nach einem der vorhergehenden Ansprüche, wobei die Speicherschaltung (236) als mehrmals beschreibbarer Speicher ausgeführt ist.

5. Sensor nach einem der vorhergehenden Ansprüche, wobei die physiologischen Daten die Sauerstoffsättigung des Blutes umfassen.

6. Sensor nach Anspruch 5, wobei die physiologischen Daten die Pulsfrequenz umfassen.

7. Sensor nach einem der vorhergehenden Ansprüche, wobei die physiologischen Daten vor dem Speichern auf der Speicherschaltung (236) komprimiert werden.

8. Sensor nach Anspruch 7, wobei die physiologischen Daten entweder durch Differenzkodierung oder Lauflängenkodierung komprimiert werden.

9. Sensor nach Anspruch 7, wobei die physiologischen Daten beim Speichern auf der Speicherschaltung dekomprimiert werden.

10. Sensor nach einem der vorhergehenden Ansprüche, wobei die physiologischen Daten unterabgetastet werden, um für jeden Zeitraum einen Datenabtastwert bereitzustellen, wobei ein Zeitraum ein vorbestimmter Zeitabschnitt ist, der zum Teil auf Basis von Eigenschaften der gespeicherten physiologischen Daten ausgewählt wird.

11. Sensor nach einem der vorhergehenden Ansprüche, wobei die Speicherschaltung (236) Informationen bereitstellt, die anzeigen, wenn die Speicherschaltung (236) voll ist.

12. Sensor nach einem der vorhergehenden Ansprüche, wobei die Speicherschaltung (236) außerdem Informationen eines Zeitpunktes, der zu jedem der spezifischen Abtastwerte der physiologischen Daten gehört, speichert.

13. Sensor nach einem der vorhergehenden Ansprüche, wobei die Speicherschaltung (236) außerdem Informationen speichert, die anzeigen, wenn der Sensor nicht mit einem Monitor (110) verbunden ist.

14. Sensor nach einem der vorhergehenden Ansprüche, weiterhin aufweisend:
mindestens eine Lichtquelle (230), wobei jede Lichtquelle ausgewählt ist, mit einer unterschiedlichen Wellenlänge zu arbeiten;
mindestens einen Fotodetektor (240), der betrieben wird um Licht, das von der mindestens einen Lichtquelle ausgesendet wird, zu empfangen; und
eine mit der Speicherschaltung gekoppelte Schnittstellenschaltung (238), wobei die Schnittstellenschaltung die Datenübertragung von und zu der Speicherschaltung koordiniert.

15. Verfahren zum Speichern physiologischer Daten, aufweisend:
Erfassen mindestens eines Signals, das einen physiologischen Zustand anzeigt, über Mittel (240) eines physiologischen Sensors (130) nach einem der vorhergehenden Ansprüche;
Aufbereiten des mindestens einen Signals zur Erzeugung von Datenabtastwerten;
Verarbeiten der Datenabtastwerte in einem von dem Sensor (130) separaten Fernmonitor (110) zur Erzeugung physiologischer Daten, wobei die physiologischen Daten den physiologischen Zustand beschreiben; und
Speichern der physiologischen Daten innerhalb des Speichers (236), der innerhalb des Sensors (130) separat von den Mitteln (240) und separat von dem Fernmonitor (110) angeordnet ist.

16. Verfahren nach Anspruch 15, wobei die Datenübertragung zwischen dem Speicher (236) und dem Fernmonitor (110) über separate Signalleitungen (270) ausgeführt wird.

17. Verfahren nach einem der vorhergehenden Ansprüche 15 oder 16, weiterhin aufweisend: Kodieren der physiologischen Daten zur Erzeugung komprimierter physiologischer Daten.

18. Verfahren nach einem der vorhergehenden Ansprüche 15-17, weiterhin aufweisend:
Abtasten oder Vorverarbeiten der physiologischen Daten zur Erzeugung komprimierter physiologischer Daten.

19. Verfahren nach einem der vorhergehenden Ansprüche 15-18, wobei die physiologischen Daten die Sauerstoffsättigung des Blutes umfassen.

20. Physiologisches Testinstrument (100), aufweisend:
einen Fernmonitor (110), aufweisend:
- eine Aufbereitungsschaltung (250, 252, 254, 256) zum Empfang eines elektrischen Signals, wobei das elektrische Signal zur Bereitstellung abgetasteter Daten verarbeitet wird; und
- eine Verarbeitungsschaltung (262) zur Verarbeitung der abgetasteten Daten zur Bereitstellung physiologischer Daten, wobei die physiologischen Daten einen physiologischen Zustand eines Patienten anzeigen; und
einen physiologischen Sensor (130), der an einen Fernmonitor (110) angeschlossen werden kann, wobei der physiologische Sensor (130) ein physiologischer Sensor nach einem der vorhergehenden Ansprüche 1-14 ist, wobei das Mittel (240) zum Erhalt von Signalen aufweist:
- mindestens eine Lichtquelle (230), wobei jede Lichtquelle ausgewählt ist, mit einer unterschiedlichen Wellenlänge zu arbeiten;
- mindestens einen Fotodetektor (240), der betrieben wird um Licht, das von der mindestens einen Lichtquelle (230) ausgesendet wird, zu empfangen.

21. Testinstrument nach Anspruch 20, wobei der Fernmonitor (110) außerdem Mittel (268) aufweist, die auf eine Benutzereingabe reagieren, zur Übertragung der mindestens einigen der physiologischen Daten an die Speicherschaltung (236) als Reaktion auf die Benutzereingabe.

22. Testinstrument nach Anspruch 20 oder 21, wobei der Fernmonitor (110) außerdem Mittel (268) aufweist, die auf ein Auftreten einer Sauerstoffentsättigung reagieren, zur Übertragung der mindestens einigen der physiologischen Daten an die Speicherschaltung (236) als Reaktion auf ein Auftreten einer Sauerstoffentsättigung bei dem Patienten.

23. Testinstrument nach einem der vorhergehenden Ansprüche 20-22, wobei der Fernmonitor (110) außerdem Mittel (268) aufweist, die auf ein Überschreiten eines Schwellenwertes reagieren, zur Übertragung der mindestens einigen der physiologischen Daten an die Speicherschaltung, wenn eine Sauerstoffsättigung des Patienten um mehr als einen vorbestimmten Betrag von einer vorherigen Sauerstoffsättigung des Patienten abweicht.

24. Testinstrument nach einem der vorhergehenden Ansprüche 20-23, wobei der Fernmonitor (110) außerdem einen Encoder (262) aufweist, der mit der Verarbeitungsschaltung gekoppelt ist, wobei der Encoder die physiologischen Daten zur Bereitstellung komprimierter physiologischer Daten kodiert.

25. Testinstrument nach Anspruch 24, wobei der Fernmonitor (110) außerdem einen Decoder (262) aufweist, der komprimierte physiologische Daten von der Speicherschaltung empfängt und die Daten dekodiert.

26. Testinstrument nach einem der vorhergehenden Ansprüche 20-25, wobei die physiologischen Daten die Sauerstoffsättigung des Blutes umfassen.

27. Testinstrument nach einem der vorhergehenden Ansprüche 20-26, wobei das Testinstrument ein Oximetersystem zur Speicherung und Bereitstellung historischer Sättigungsdaten eines Patienten ist, aufweisend:
mindestens zwei Lichtquellen (230) zur Lichtübertragung durch den Patienten hindurch, wobei die Lichtquellen mit unterschiedlichen Wellenlängen arbeiten, wobei der Fotodetektor angepasst ist, optische Signale von den Lichtquellen zu empfangen und angepasst ist, elektrische Signale bereitzustellen, die für die empfangenen optischen Signale kennzeichnend sind, wobei die von der Verarbeitungsschaltung erzeugten physiologischen Daten Sättigungsdaten sind; und außerdem aufweisend eine Schaltung, die die Anzeige der Sättigungsdaten steuert.

## Revendications

1. Capteur physiologique pouvant être raccordé à un moniteur distant (110), le capteur physiologique (130) comprenant :
un moyen (240) servant à obtenir des signaux d'un patient (234) révélateurs de la condition physiologique du patient ;
un moyen (240) servant à envoyer des signaux au moniteur distant ;
un circuit de mémoire (236) intégré dans le capteur et situé à part du moniteur distant ; et un circuit d'interface (238) couplé au circuit de mémoire (236), dans lequel le circuit d'interface (238) facilite le transfert de données vers et à partir du circuit de mémoire (236),
**caractérisé en ce que** le circuit de mémoire (236) est à part dans le capteur et est électriquement isolé du moyen (240) destiné à obtenir des signaux et du moyen (240) destiné à envoyer les signaux ; et adapté pour le transfert des données physiologiques du patient dérivées des signaux et révélatrices de la condition physiologique entre le moniteur distant (110) et le circuit de mémoire (236), lorsque le moniteur distant (110) le demande.

2. Capteur selon la revendication 1, dans lequel l'interface (238) peut être couplée au moniteur distant (110) via des circuits d'acheminement des signaux distincts (270).

3. Capteur selon l'une des revendications précédentes, dans lequel le circuit de mémoire (236) est implanté comme mémoire non réinscriptible, mémoire FLASH, mémoire vive (RAM), mémoire réinscriptible, ou seulement mémoire morte effaçable et programmable électriquement (EEPROM).

4. Capteur selon l'une des revendications précédentes, dans lequel le circuit de mémoire (236) est implanté comme mémoire à multiples réinscriptions.

5. Capteur selon l'une des revendications précédentes, dans lequel les données physiologiques comprennent des données de saturation en oxygène du sang.

6. Capteur selon la revendication 5, dans lequel les données physiologiques incluent des données de fréquence du pouls.

7. Capteur selon l'une des revendications précédentes, dans lequel les données physiologiques sont comprimées avant stockage sur le circuit de mémoire (236).

8. Capteur selon la revendication 7, dans lequel les données physiologiques sont comprimées en utilisant soit un codage différentiel ou un encodage de plage.

9. Capteur selon la revendication 7, dans lequel les données physiologiques sont décomprimées une fois stockées sur le circuit de mémoire.

10. Capteur selon l'une des revendications précédentes, dans lequel les données physiologiques sont sous-échantillonnées pour fournir un échantillon de données pour chaque époque, dans lequel une époque est une période de temps prédéterminée choisie, en partie, sur la base des caractéristiques des données physiologiques stockées.

11. Capteur selon l'une des revendications précédentes, dans lequel le circuit de mémoire (236) fournit des informations qui indiquent quand le circuit de mémoire (236) est plein.

12. Capteur selon l'une des revendications précédentes, dans lequel le circuit de mémoire (236) stocke en outre des informations d'un moment associé à chaque échantillon spécifique des données physiologiques.

13. Capteur selon l'une des revendications précédentes, dans lequel le circuit de mémoire (236) stocke en outre des informations révélatrices de la déconnexion du capteur d'un moniteur (110).

14. Capteur selon l'une des revendications précédentes, comprenant en outre :
au moins une source lumineuse (230), chaque source lumineuse est choisie pour fonctionner à une longueur d'onde différente ;
au moins un photo-détecteur (240) utilisé pour recevoir la lumière émise par la au moins une source lumineuse ; et
un circuit d'interface (238) couplé au circuit de mémoire, dans lequel le circuit d'interface coordonne le transfert de données vers et à partir du circuit de mémoire.

15. Procédé servant à stocker des données physiologiques comprenant :
la détection via un moyen (240) d'un capteur physiologique (130) selon l'une quelconque des revendications précédentes d'au moins un signal révélateur d'une condition physiologique ;
le conditionnement du au moins un signal détecté pour générer des échantillons de données ;
le traitement des échantillons de données dans un moniteur distant (110), séparé du capteur (130) pour générer des données physiologiques, dans lequel les données physiologiques décrivent la condition physiologique ; et
le stockage des données physiologiques dans la mémoire (236) situé à part dans le capteur (130) à partir du moyen (240) et à part du moniteur distant (110).

16. Procédé selon la revendication 15, dans lequel le transfert de données entre la mémoire (236) et le moniteur distant (110) est exécuté par les circuits d'acheminement des signaux distincts (270).

17. Procédé selon l'une des revendications précédentes 15 ou 16 comprenant en outre : le codage des données physiologiques pour générer des données physiologiques comprimées.

18. Procédé selon l'une des revendications précédentes 15 à 17 comprenant en outre : le prélèvement ou le prétraitement des données physiologiques pour générer des données physiologiques comprimées.

19. Procédé selon l'une des revendications précédentes 15 à 18, dans lequel les données physiologiques incluent des données de saturation en oxygène du sang.

20. Instrument d'essais physiologiques (100) comprenant :
un moniteur distant (110) comprenant :
- des circuits de conditionnement (250, 252, 254, 256) servant à recevoir un signal électrique et à traiter le signal électrique pour fournir des données prélevées ; et
- des circuits de traitement (262) servant à traiter les données prélevées pour fournir des données physiologiques, dans lequel les données physiologiques sont révélatrices d'une condition physiologique d'un patient ; et
un capteur physiologique (130) pouvant être raccordé au moniteur distant (110), dans lequel le capteur physiologique (130) est un capteur physiologique selon l'une quelconque des revendications 1 à 16, dans lequel le moyen (240) servant à obtenir des signaux comprend :
- au moins une source lumineuse (230), chaque source lumineuse est choisie pour fonctionner à une longueur d'onde différente ; et
- au moins un photo-détecteur (240) utilisé pour recevoir la lumière émise par la au moins une source lumineuse (230).

21. Instrument d'essais selon la revendication 20, dans lequel le moniteur distant (110) inclut en outre un moyen (268) sensible aux données entrées de l'utilisateur pour transférer au moins certaines des données physiologiques sur le circuit de mémoire (236) en réponse aux données entrées de l'utilisateur.

22. Instrument d'essais selon la revendication 20 ou 21, dans lequel le moniteur distant (110) inclut en outre un moyen (268) sensible à un événement de désaturation en oxygène pour transférer au moins certaines des données physiologiques sur le circuit de mémoire (236) en réponse à un événement de désaturation de l'oxygène du patient.

23. Instrument d'essais selon l'une des revendications précédentes 20 à 22, dans lequel le moniteur distant (110) inclut en outre un moyen (268) sensible à un croisement seuil pour transférer au moins certaines des données physiologiques sur le circuit de mémoire quand une saturation en oxygène du patient diffère de plus d'une quantité prédéterminée d'une saturation en oxygène précédente du patient.

24. Instrument d'essais selon l'une des revendications précédentes 20 à 23, dans lequel le moniteur distant (110) inclut en outre un encodeur (262) couplé aux circuits de traitement, dans lequel l'encodeur code les données physiologiques pour fournir des données physiologiques comprimées.

25. Instrument d'essais selon la revendication 24, dans lequel le moniteur (110) inclut en outre un décodeur (262) qui reçoit des données physiologiques comprimées du circuit de mémoire et décode les données.

26. Instrument d'essais selon l'une des revendications précédentes 20 à 25, dans lequel les données physiologiques incluent des données de saturation en oxygène du sang.

27. Instrument d'essais selon l'une des revendications précédentes 20 à 26 ; dans lequel l'instrument d'essais est un système d'oxymètre servant à stocker et à fournir l'historique des données de saturation d'un patient comprenant :
au moins deux sources lumineuses (230) servant à transmettre la lumière à travers le patient, dans lequel les sources lumineuses fonctionnent à différentes longueurs d'onde ; dans lequel le photo-détecteur est adapté pour recevoir des signaux optiques à partir des sources lumineuses et adapté pour fournir des signaux électriques révélateurs des signaux optiques reçus ; dans lequel les données physiologiques générées par les circuits de traitement sont des données de saturation ; et comprenant en outre des circuits qui dirigent l'affichage des données de saturation.
